(19) Europäisches Patentamt
European Patent Office
Office européen des brevets

(11) **EP 2 578 087 B1**

(12) **EUROPEAN PATENT SPECIFICATION**

(45) Date of publication and mention
of the grant of the patent:
**03.12.2014 Bulletin 2014/49**

(51) Int Cl.:
*A23K 1/18* (2006.01)       *A23K 1/175* (2006.01)
*A61K 31/232* (2006.01)     *A23K 1/16* (2006.01)
*A61K 31/231* (2006.01)

(21) Application number: **11786528.7**

(22) Date of filing: **18.05.2011**

(86) International application number:
**PCT/JP2011/061358**

(87) International publication number:
**WO 2011/148829 (01.12.2011 Gazette 2011/48)**

(54) **PET FOOD**

HAUSTIERFUTTER

ALIMENT POUR ANIMAUX DE COMPAGNIE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **25.05.2010 JP 2010119053
25.05.2010 JP 2010119052**

(43) Date of publication of application:
**10.04.2013 Bulletin 2013/15**

(73) Proprietor: **Kao Corporation
Chuo-Ku
Tokyo 103-8210 (JP)**

(72) Inventors:
• **MIZUUCHI, Sachiyo
Haga-gun
Tochigi 321-3497 (JP)**
• **UMEDA, Tomoshige
Haga-gun
Tochigi 321-3497 (JP)**

(74) Representative: **Vossius & Partner
Siebertstrasse 4
81675 München (DE)**

(56) References cited:
JP-A- 2007 110 916       JP-A- 2009 509 498
US-A1- 2009 148 560

## Description

Field of the Invention

The present invention relates to a pet food including an unsaturated fatty acid-containing oil and fat.

Background of the Invention

The number of pets has been increasing with the current popularity of pets. Along with the trend, the number of pets suffering from diseases similar to human adult diseases, such as obesity, diabetes, andliverdiseases, duetoaging, lack of exercise and nutrient excess of the pets has been increasing. In addition, there are many dogs and cats which get fat because of hormone imbalance after sterilization. Many pet foods for preventing such obesity and body weight gain have been developed. For example, a pet food including an oil and fat having an increased unsaturated fatty acid content has been proposed.

Such pet food contains a large amount of the unsaturated fattyacid, and hence is easily oxidized in some storage environments, which may affect odor and palatability of the pet food. In view of the foregoing, the applicant of the present invention has proposed improvement of long-term storage stability by a technology of blending a vitamin C derivative in a pet food having a high mineral content (Patent Document 1) and a technology of incorporating a naturally-occurring antioxidant and controlling contents of an iron ion and a copper ion in a pet food (Patent Document 2).

In addition, for example, there have been proposed antioxidation systems for foods, such as a combination of a glyceride containing a medium-chain fatty acid as a constituent fatty acid and an antioxidant (Patent Document 3), a combination of a bayberry extract, a water-soluble antioxidant, and a lipophilic antioxidant (Patent Document 4), and a combination of a mushroom mycelium culture filtrate and an antioxidant (Patent Document 5).

Prior Art Document

Patent Document

[

Patent Document 1] JP-A-2005-204659
[Patent Document 2] JP-A-2007-110916
[Patent Document 3] JP-A-2004-115758
[Patent Document 4] JP-A-2007-185138
[Patent Document 5] JP-A-7-59548

Summary of the Invention

The present invention provides a pet food, including the following components (A) to (D):

(A) an oil and fat including, as a constituent fatty acid, an unsaturated fatty acid having two or more double bonds;
(B) a mineral component selected from iron, an iron compound, copper, and a copper compound;
(C) one or more kinds selected from citric acid and a salt thereof; and
(D) one or more kinds selected from a lecithin and a rosemary extract, wherein the total amount of the component (C) is 10 times or more that of the component (B) in terms of molar ratio.

The present invention also provides a method of preventing oxidation of a composition including (A) an oil and fat containing, as a constituent fatty acid, an unsaturated fatty acid having two or more double bonds and (B) a mineral component selected from iron, an iron compound, copper, and a copper compound, the method including blending, in the composition, (C) one or more kinds selected from citric acid and a salt thereof and (D) one or more kinds selected from a lecithin and a rosemary extract.

Brief Description of the Drawings

[FIG. 1] A graph showing results of evaluation of antioxidative activities of various antioxidants against compositions including an oil and fat and a mineral component by a Rancimat test method.

[FIG. 2] A graph showing time-course changes in electrical conductivity of a sample.
[FIG. 3] A drawing showing effects of the amount and kind of a lecithin on Rancimat rising time.
[FIG. 4] A graph showing results of evaluation of antioxidative activities of various antioxidants against compositions including an oil and fat and a mineral component by the Rancimat test method.
[FIG. 5] A graph showing effects of blending of a rosemary extract in addition to citric acid and/or a salt thereof on Rancimat rising time.

Embodiment for Carrying out the Invention

With increasing health consciousness for pets in recent years, there has been a demand for a pet food containing a large amount of an oil and fat including, as a constituent fatty acid, a fatty acid having many unsaturated bonds in its molecule, such as an $\omega$3 fatty acid or an $\omega$6 fatty acid, which is expected to have various physiological effects. However, the fatty acid is very easily oxidized and is difficult to exert the physiological effects for a long period of time. Therefore, it has been required to achieve long-term storage stability in such pet food including an oil and fat containing, as a constituent fatty acid, a highly unsaturated fatty acid.

Therefore, the inventors of the present invention studied long-term storage stability of a pet food including an oil and fat including, as a constituent fatty acid, an unsaturated fatty acid having two or more double bonds, and found that, when a metal salt necessary for a pet food, in particular, copper or iron is blended with an unsaturated fatty acid-containing oil and fat, oxidation of the oil and fat is promoted and thus a pet food which contains both of them and is stable against oxidation cannot be obtained. Accordingly, the inventors made various studies to improve the oxidation stability, and found that while oxidation of a pet food including the unsaturated fatty acid-containing oil and fat and the metal salt cannot be prevented sufficiently by singly blending catechin, vitamin E, lecithin, vitamin C or carotene known to have antioxidative effects, oxidation of the pet food can significantly be prevented by blending citric acid or a salt thereof in a predetermined amount.

In addition, the inventors of the present invention studied to further improve the stabilization effect against oxidation of the pet food by blending citric acid or a salt thereof, and as a result, surprisingly, found that stability of the pet food including the unsaturated fatty acid-containing oil and fat and the metal salt can be significantly improved by blending the lecithin or rosemary extract, which has substantially no antioxidative effect when added singly, together with a certain amount or more of citric acid or a salt thereof, thus completing the present invention.

The pet food of the present invention is very excellent in long-term storage stability. Therefore, for example, in the case where an oil and fat containing, as a constituent fatty acid, a large amount of a highly unsaturated fatty acid such as $\omega$3 or $\omega$6 is blended therein, the expected physiological function can be expressed for a long period of time. In addition, it is possible to effectively suppress a change in the odor and deterioration of the palatability, caused by oxidation of the fatty acid.

Preferred exemplary embodiments of the present invention are as follows.

[1] A pet food, including the following components (A) to (D):

(A) an oil and fat including, as a constituent fatty acid, an unsaturated fatty acid having two or more double bonds;
(B) a mineral component selected from iron, an iron compound, copper, and a copper compound;
(C) one or more kinds selected from citric acid and a salt thereof; and
(D) one or more kinds selected from a lecithin and a rosemary extract, wherein the total amount of the component (C) is 10 times or more that of the component (B) in terms of molar ratio.

[2] A method of preventing oxidation of a composition including (A) an oil and fat containing, as a constituent fatty acid, an unsaturated fatty acid having two or more double bonds and (B) a mineral component selected from iron, an iron compound, copper, and a copper compound,
the method including blending, in the composition, (C) one or more kinds selected from citric acid and a salt thereof and (D) one or more kinds selected from a lecithin and a rosemary extract.
[3] The pet food or method according to the above-mentioned item [1] or [2], wherein the component (D) includes any one of the lecithin and the rosemary extract.
[4] The pet food or method according to the above-mentioned item [1] or [2], wherein the component (D) includes both of the lecithin and the rosemary extract.
[5] The pet food or method according to any one of the above-mentioned items [1] to [4], wherein the component (A) is an oil and fat containing, as a constituent fatty acid, 20 to 90 mass% of an unsaturated fatty acid having two or more double bonds.
[6] The pet food or method according to any one of the above-mentioned items [1] to [5], wherein the component (A) is an oil and fat containing, as a constituent fatty acid, 3 mass% or more of an unsaturated fatty acid having three or more double bonds.

[7] The pet food or method according to any one of the above-mentioned items [1] to [6], wherein the content of the component (A) in the pet food or the composition is 5 to 80 mass%.

[8] The pet food or method according to any one of the above-mentioned items [1] to [7], wherein the mineral component (B) is contained as iron or an iron compound in an amount of 0.00001 to 1 mass% in terms of iron in the pet food or the composition.

[9] The pet food or method according to any one of the above-mentioned items [1] to [8], wherein the mineral component (B) is contained as copper or a copper compound in an amount of 0.00001 to 0.1 mass% in terms of copper in the pet food.

[10] The pet food or method according to any one of the above-mentioned items [1] to [9], wherein the component (C) is one or more kinds selected from citric acid and an alkali metal citrate.

[11] The pet food or method according to any one of the above-mentioned items [1] to [10], wherein the total amount of the component (C) is 10 to 60 times that of the component (B) in terms of molar ratio.

[12] The pet food or method according to any one of the above-mentioned items [1] to [11], wherein the component (D) includes phosphatidylethanolamine, and the content thereof in the pet food or the composition is 0.03 mass% or more.

[13] The pet food or method according to any one of the above-mentioned items [1] to [12], wherein the content of the lecithin as the component (D) in the pet food or the composition is 0.06 to 6.0 mass%.

[14] The pet food or method according to any one of the above-mentioned items [1] to [13], wherein the content of the rosemary extract as the component (D) in the pet food or the composition is 0.004 mass% or more.

[15] The pet food or method according to any one of the above-mentioned items [1] to [14], wherein the pet food or the composition further includs (E) at least one kind of polyphenol selected from catechins, curcumin, quercetin, and myricitrin.

[16] The pet food or method according to the above-mentioned item [15], wherein the component (E) is catechins.

[17] The pet food or method according to the above-mentioned item [15] or [16], wherein the content of the component (E) in the pet food or the composition is 0.001 to 35 mass%.

[18] The pet food or method according to any one of the above-mentioned items [1] to [17], wherein the pet food or the composition has a pH of 3.5 to 7.0.

[19] The pet food or method according to any one of the above-ment ioned items [1] to [18], wherein the pet food or the composition is dry-type.

The pet food of the present invention contains (A) an oil and fat including, as a constituent fatty acid, an unsaturated fatty acid having two or more double bonds (hereinafter, also simply referred to as highly unsaturated fatty acid). The content of the highly unsaturated fatty acid in the constituent fatty acid of the oil and fat is preferably 20 to 90 mass% (hereinafter, simply referred to as "%"), more preferably 23 to 80%, even more preferably 25 to 70% in view of health of the skin and hair of a pet. In addition, the content of the oil and fat containing, as a constituent fatty acid, the highly unsaturated fatty acid in the pet food is preferably 1 to 45%, more preferably 2 to 45%, even more preferably 2.5 to 40%, even more preferably 3 to 35%. Here, suitable examples of the highly unsaturated fatty acid include a highly unsaturated fatty acid having 18 to 24 carbon atoms. Examples of the unsaturated fatty acid having two double bonds include linoleic acid. Further, the oil and fat preferably contains a large amount of an unsaturated fatty acid having three or more double bonds from the viewpoint that various physiological effects can be expected to be exerted. The unsaturated fatty acid is preferably $\alpha$-linolenic acid, $\gamma$-linolenic acid, arachidonic acid, eicosapentaenoic acid (EPA), or docosahexaenoic acid (DHA). The content of the fatty acid in the constituent fatty acid of the oil and fat as the component (A) is preferably 3.0% or more, more preferably 3.5% or more, even more preferably 4.0% or more.

It should be noted that, as the constituent fatty acid of the oil and fat as the component (A), an unsaturated fatty acid having one double bond and a saturated fatty acid may be contained in addition to the highly unsaturated fatty acid. Here, examples of the unsaturated fatty acid having one double bond include an unsaturated fatty acid having 16 to 24 carbon atoms. Of those, oleic acid, palmitoleic acid, and the like are preferred. In addition, examples of the saturated fatty acid include a saturated fatty acid having 12 to 18 carbon atoms. Of those, palmitic acid and stearic acid are suitable.

In the present invention, the pet food may include an oil and fat other than the component (A), i.e., an oil and fat which does not contain the highly unsaturated fatty acid as a constituent fatty acid. The content of all the oils and fats in the pet food of the present invention is preferably 6 to 50%, more preferably 6 to 45%, even more preferably 6 to 40%, even more preferably 8 to 35% in view of palatability and removal of unpleasant odor of the pet food. Further, the content of the unsaturated fatty acid having two or more double bonds in the constituent fatty acids of all the oils and fats in the pet food is preferably 5% to 80%, more preferably 10 to 75%, even more preferably 15 to 70%, even more preferably 20 to 65%.

Examples of the oils and fats used in the pet food of the present invention include safflower oil, olive oil, cotton seed oil, corn oil, rapeseed oil, soybean oil, palm oil, sunflower oil, linseed oil, sesame oil, lard, beef tallow, fish oil, milk fat, coconut oil, copra oil, rice bran oil, perilla oil, perilla seed oil, evening primrose oil, and hemp seed oil which contain the highly unsaturated fatty acid as a constituent fatty acid. Of those, linseed oil, fish oil, perilla oil, perilla seed oil, evening primrose oil, and hemp seed oil which contain an unsaturated fatty acid having three or more double bonds as a constituent fatty acid are preferred. However, the oils and fats include not only the materials blended as the oils and fats but also another plant or animal material containing an oil and fat. In addition, the oils and fats may contain diglycerides.

The pet food of the present invention further contains a mineral component selected from iron, an iron compound, copper and a copper compound as the component (B). Examples of the iron compound include iron sulfate, ferric chloride, ferrous fumarate, ferrous carbonate, and iron oxide. The content of the iron compound in the pet food is preferably 0.00001 to 1%, more preferably 0.00005 to 0.3%, even more preferably 0.0001 to 0.1% in terms of iron, which fall within an acceptable range for blending in the pet food.

Examples of the copper compound include copper sulfate, copper chloride, copper oxide, copper carbonate, copper sulfide, and copper chlorophyll. The content of the copper compound in the pet food is preferably 0.00001 to 0.1%, more preferably 0.00005 to 0.03%, even more preferably 0.0001 to 0.01% in terms of copper, which fall within an acceptable range for blending in the pet food.

Of those mineral components, the iron or iron compound and the copper or copper compound are preferably used in combination. In the present invention, the total content of the mineral components is preferably 0.0005 to 1%, more preferably 0.0005 to 0.5%, even more preferably 0.001 to 0.1% in terms of mineral (in terms of iron and in terms of copper).

The pet food of the present invention may include, as the mineral components, manganese, cobalt, calcium, phosphorus, sodium, potassium and the like in addition to iron and copper. The mineral components are blended, for example, as manganese oxide, cobalt carbonate, calcium carbonate, calcium phosphate, sodium chloride and potassium chloride.

The pet food of the present invention further includes, as the component (C), one or more kinds selected from citric acid and a salt thereof, and the total amount of the component (C) is 10 times or more that of the component (B) in terms of molar ratio. Here, examples of the salt of citric acid include an alkali metal salt and an alkaline earth metal salt, and in particular, an alkali metal salt is preferred. The alkali metal salt means a normal salt (trialkali metal salt), a monohydrogen dialkali metal salt, and a dihydrogen alkali metal salt of citric acid. Examples of the alkali metal salt of citric acid include a sodium salt and a potassium salt, and in particular, a sodium salt is preferred. It should be noted that, in this description, sodium citrate refers to trisodium citrate (dihydrate) unless otherwise specified.

When the total amount of citric acid and a salt thereof is 10 times or more that of the metal as the component (B) in terms of molar ratio, the antioxidation effect obtained by combined use of citric acid and/or a salt thereof and a lecithin or a rosemary extract is synergistically improved. The total amount of the component (C) is preferably 11 times or more, more preferably 12 times or more, even more preferably 15 times or more, even more preferably 18 times or more that of the component (B) in terms of molar ratio. In addition, the total amount of citric acid or a salt thereof is preferably 60 times or less that of the component (B) in view of palatability.

In addition, the pet food preferably includes the component (C) in an amount of 0.5% or more in terms of citric acid. The phrase "in terms of citric acid" as used herein refers that, in the case where the pet food includes, as the component (C), citric acid and an alkali metal salt of citric acid (hereinafter, also referred to as citric acids), or in the case where the component (C) includes only an alkali metal salt of citric acid, the mass is converted by replacing the alkali metal by hydrogen and calculating a molecular weight of citric acid.

The pet food of the present invention includes the citric acids in an amount of preferably 0.7% or more, more preferably 0.75% or more, even more preferably 0.9% or more, even more preferably 1.0% or more in terms of citric acid in view of storage stability and palatability. The upper limit of the content of the citric acids is preferably 5.0%, more preferably 4.0% in view of palatability and odor during storage.

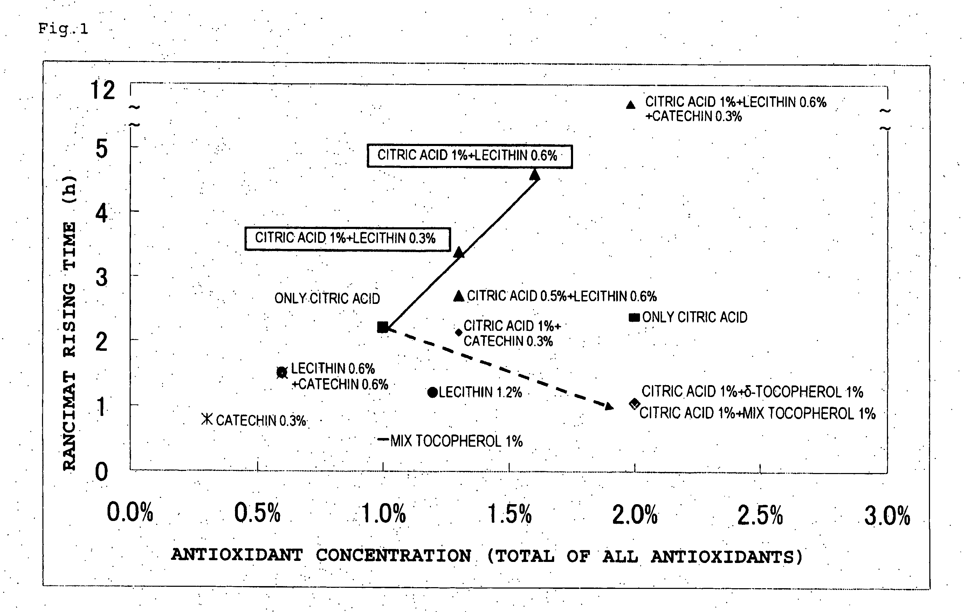

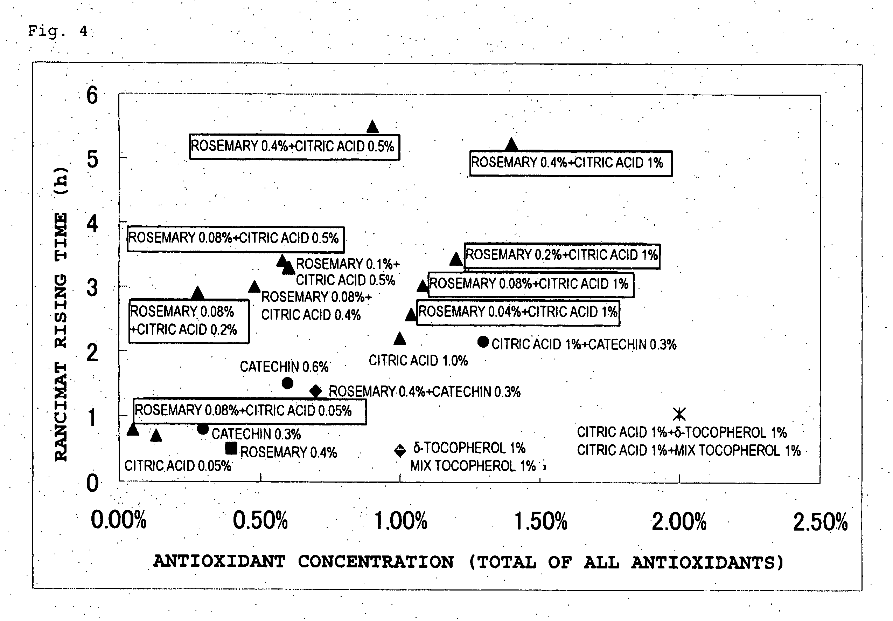

The pet food of the present invention includes, as the component (D), one or more kinds selected from a lecithin and a rosemary extract, together with the component (C). In the case where the lecithin or rosemary extract is used singly in a composition including the mineral component and the oil and fat containing the highly unsaturated fatty acid as a constituent fatty acid like the present invention, the effect is low and is lower than the antioxidative activity in a composition including no mineral component (see FIGS. 1 and 4). On the other hand, in a composition including citric acid singly in an amount of 1.0% or more, the antioxidative activity is raised to the level of the composition including no mineral component. The present invention has revealed that the antioxidative activity can be improved by using both of the citric acid and the lecithin or rosemary extract to a level unimaginable from the effect obtained by using one of the citric acid and the lecithin or rosemary extract. In actuality, in the case of using both the citric acid and mix tocopherol which is another generally-known antioxidant or $\delta$-tocopherol which is known to have a very high effect, a phenomenon where the antioxidative activity decreased compared with that in case of use of only citric acid was observed (FIGS. 1 and 4). That is, it can be said that improvement of the antioxidative activity is a phenomenon specific to a combination of citric acid and the lecithin or rosemary extract.

The lecithin means a phosphatidylcholine in a limited sense but means a glycerophospholipid in a broad sense.

Phosphatidylethanolamine which is suitably used in the present invention is one of the glycerophospholipids and included in a broad-sense lecithin. In the case where the lecithin is used for commercial or industrial use, the lecithin means a broad-sense glycerophospholipid, and is usually a mixture including the glycerophospholipid as a major component.

Examples of the lecithin as the component (D) used in the present invention include soybean lecithin, powder soybean lecithin, egg yolk lecithin, hydrogenated soybean lecithin, hydrogenated egg yolk lecithin, rapeseed lecithin, corn lecithin, bovine brain lecithin and bovine liver lecithin. In addition, soybean lecithin components generally used in food and feed include phosphatidylcholine (PC), phosphatidylethanolamine (PE), phosphatidylinositol (PI), and phosphatidic acid (PA) in descending order of content, the components are independently considered as a lecithin in the present invention. In addition, examples of the lecithin include PC-DHA in which DHA is bound as a constituent fatty acid of PC. Of those lecithins, PE was found to have high antioxidative activity. Therefore, the mixture desirably has a high PE content and desirably contains a large amount of the highly unsaturated fatty acid. Soybean lecithin, powder soybean lecithin, hydrogenated soybean lecithin, and egg yolk lecithin are more preferred from the viewpoint of taste and flavor, odor, and cost. In particular, soybean lecithin is preferred from the viewpoint of onset of the effect and cost.

In view of the antioxidative effect, the content of the lecithin as the component (D) is preferably 0.1% or more, more preferably 0.3% or more, even more preferably 0.5% or more relative to the oil and fat as the component (A). From the viewpoint of a higher antioxidative effect and taste and flavor, the content is preferably 0.6% or more and 20.0% or less, more preferably 1.0% or more and 15.0% or less.

It should be noted that, in the present invention, the amount of the lecithin as the component (D) refers to an amount in terms of phospholipid which is acetone-insoluble matter, and the amount in terms of phospholipid can be measured by a method described in "Standard Methods for the Analysis of Fats, OilsandRelatedMaterials, 5.3.3.1-86 Phospholipid Phosphorus Composition" edited by Japan Oil Chemists' Society. For example, soybean lecithin which is generally used in foods contains acetone-soluble matter such as a triglyceride, and such component is excluded. In many cases, the amount of the lecithin which is the acetone-insoluble matter accounts for about 60% of a lecithin source (for example, a preparation commercially available as soybean lecithin). In addition, the content of a phospholipid in the pet food can be preferably determined by extracting an oil and fat from the pet food with a solvent of chloroform/methanol and quantifying the content of phosphorous in the oil and fat. The content of phosphorous in the oil and fat can be quantified by a colorimetric method in accordance with "Standard Methods for the Analysis of Fats, Oils and Related Materials" (Japan Oil Chemists' Society, 2.4.11-1996) (conversion factor from the amount of phosphorus: 25.4). The value of the phospholipid content determined by the quantification method is in excellent agreement with the amount in terms of the phospholipid which is the acetone-insoluble matter. Further, the content of the lecithin as the component (D) is preferably 0.06 to 6.0%, more preferably 0.1 to 3%, even more preferably 0.2 to 2.0%, in the total amount of the pet food of the present invention from the viewpoint of an antioxidative effect and taste and flavor. In addition, the content of PE as the component (D) is preferably 0.03% or more, more preferably 0.04% or more in the pet food, from the viewpoint ofahighantioxidativeeffect. Moreover, the content of the lecithin as the component (D) is preferably 0.5% or more and 75% or less, more preferably 0.5% or more and 50% or less relative to the highly unsaturated fatty acid which is a component of the oil and fat as the component (A) from the viewpoint of a high antioxidative effect.

The rosemary extract as the component (D) used in the present invention is a water and/or organic solvent extract of leaves of rosemary which is a plant belonging to *Lamiaceae,* and generally has an odor specific to rosemary which is herb. More specifically, the extract is obtained by drying and pulverizing leaves of rosemary, and subjecting the resultant product to extraction with, for example, water, hot water, hexane, ethanol, acetone, ethyl acetate, or a mixed solvent thereof. In the present invention, there may be used the above-mentioned extracts obtained with water and/or an organic solvent, oleoresin preparations obtained by formulating the extracts, or preparations including rosmanol, carsonol and isorosmanol , which are components. Particularly preferred are products obtained by subjecting the extracts to deodorization treatment by reduced pressure method, heating reduced pressure method, supercritical extraction method or column adsorption method (hereinafter, referred to as deodorized products) in view of taste and flavor. Here, as a commercially available rosemary extract, there are given, for example, Herbalox type O, Herbalox type HT-O, Herbalox type 25, Duolite NMH, and Duolite NM-1 (all of which are manufactured by Kalsec), Leomeal E and Leomeal IO (all of which are manufactured by Lion Corporation), and RM keeper (manufactured by Mitsubishi-Kagaku Foods Corporation).

The content of the rosemary extract as the component (D) is 0.04% or more in terms of extract content (no solvent) relative to the oil and fat as the component (A) in view of the antioxidative effect. From the viewpoint of a higher antioxidative effect, taste and flavor, and economy, the content is preferably 0.04% or more and 0.4% or less, more preferably 0.08% or more and 0.2% or less.

In addition, the content of the rosemary extract as the component (D) is preferably 0.004% or more, more preferably 0.008% or more, even more preferably 0.01% or more in terms of extract content (no solvent) in the total amount of the pet food of the present invention from the viewpoint of the antioxidative effect and taste and flavor.

When the pet food of the present invention further includes at least one kind of polyphenol selected from catechin, curcumin, quercetin, and myricitrin as the component (E), the antioxidative activity is further enhanced. As shown in FIG. 1, in the case where 0.3% of catechin is added to 1% of citric acid + 0.6% of lecithin (▲ in the figure), the Rancimat rising time which represents the antioxidative activity is increased up to about 3 times. Interestingly, in the case where 0.3% of catechin is added to 1% of citric acid (♦), the antioxidative activity becomes equal to or lower than that in the case of addition of only citric acid. That is, in the pet food including the components (A) and (B), improvement of the antioxidative activity by catechin is a phenomenon specific to a composition including both of citric acid and lecithin and is unimaginable from the effect obtained by using only each antioxidant.

As the polyphenol used in the present invention, a catechin is preferred because it can drastically enhance the antioxidative effect. The catechin has only to have a 3,3',4',5,7-flavanpentol skeleton, and examples thereof include catechin, epicatechin, gallocatechin, epigallocatechin, catechin gallate, epicatechin gallate, gallocatechin gallate, and epigallocatechin gallate. A tea extract may also be used as the catechin.

As a curcumin source, a turmeric extract, a ginger extract, or the like may be used. As a quercetin source, an onion skin extract or an apple skin extract. As a myricitrin source, a bayberry extract may be used.

The content of the polyphenol as the component (E) in the pet food of the present invention is preferably 0.01 to 35%, more preferably 0.02 to 5.0%, even more preferably 0.03 to 2.0% from the viewpoint of the antioxidative effect and taste and flavor. In addition, in view of achieving a higher antioxidative effect, the mass ratio (D/E) of the component (D) to the component (E) is preferably 1/1 to 100/1, more preferably 2/1 to 80/1. In the present invention, the amount of the polyphenol refers to an amount of the polyphenol which is considered to be effective. In the case of using a tea extract as the catechin, the amount refers to the total content of the above-mentioned seven catechins in the extract.

The pet food of the present invention preferably has a pH of 3.5 to 7.0. When the pH falls within the range, oxidation of the component (A) by the component (B) is more effectively suppressed, and good palatability is achieved. When the pH is 4.0 to 7.0, more preferably 4.5 to 6.8, the oxidation suppressing effect is more enhanced, and the odor during storage becomes good, which is preferred. In order to produce a pet food containing the citric acids and having a pH within the above-mentioned range, methods of adding a pH adjuster in addition to citric acid may be employed. Of those, a method involving using citric acid and a citrate in combination is preferred because adjustment of the pH is easy, and because the resultant pet food has good palatability and a well-rounded taste and flavor. In particular, the above-mentioned alkali metal citrate is preferably used as the citrate.

It should be noted that the phrase "pH of the pet food" refers to a pH measured for the pet food by the following method.

<Measurement method for pH of pet food>

10 g of the pet food were added to 60 mL of ion-exchange water (preferably ion-exchange water having an electrical conductivity at 25°C of 0.01 mS/m or less) at 45°C, and the mixture was stirred sufficiently and allowed to stand still for 1 hour. The mixture was subsequently filtered using a qualitative filter (No.1, manufactured by Advantec), and the pH of the filtrate was measured at 25°C.

It should be noted that in the case where the pet food is in a solid state, the above-mentioned stirring procedure is preferably carried out after the pet food is ground finely using a mortar.

The pet food of the present invention preferably further contains a carbohydrate. Examples of carbohydrate source include monosaccharides, oligosaccharides, polysaccharides, dietary fiber, and starches. Examples of the starches include waxy corn starch, corn starch, wheat starch, rice starch, glutinous rice starch, potato starch, sweet potato starch, tapioca starch, sago starch, and chemically treated or chemically modified products thereof, i.e., modified starches. In addition, the carbohydrate may be contained as a grain, and examples of the grain include corn, barley, wheat, rye, sorghum, rice, Japanese barnyard millet, foxtail millet, amaranthus, and quinoa. The content of the carbohydrate in the pet food is preferably 10 to 70%, more preferably 20 to 60%, even more preferably 30 to 50% inviewof economy, an obesity-preventing effect, ingestion capacity, a stool condition, and healthy and beautiful appearance.

The pet food of the present invention preferably further includes an animal or plant protein in view of the obesity-preventing effect, ingestion capacity, nutrient balance, and healthy and beautiful appearance. In view of ingestion capacity, an animal protein is more preferred. Examples of the animal protein include a milk protein such as casein. Of those, in view of obesity-preventing effect and ingestion capacity, an animal meat protein is preferred. Examples of the animal meat protein include: meat of livestock and wild animals such as cattle, swine, sheep, rabbits, and kangaroos, and by-products and processed products thereof; meat of birds such as chicken, turkey, and quail, and by-products and processed products thereof; meat of fish such as fish or white fish, and by-products and processed products thereof; and products obtained by rendering the above-mentioned materials, such as meat meal, meat born, chicken meal, poultry meal, and fish meal. Of those, chickenmeat and fishmeat are particularlypreferred in view of the obesity-preventing effect. In the case using a mixture of two or more of meat proteins, the content of the chicken meat and/or fish meat is preferably 30 to 100%, more preferably 50 to 100% relative to the whole meat material.

Preferred examples of the plant protein include a soybean protein, a wheat protein, wheat gluten, and corn gluten.

The content of the animal or plant protein in the pet food of the present invention is preferably 5 to 70%, more preferably 10 to 60%, even more preferably 15 to 40% in terms of loss on drying.

The pet foodof the present inventionpet food may further include a phytosterol. The content of the phytosterol in the pet food is preferably 0.1% or more, more preferably 0.5% or more in viewof a cholesterol-reducing effect. The upper limit of the contnet of the phytosterol has only to fall within the range of 0.1 to 30%. Here, examples of the phytosterol include free forms of $\alpha$-sitosterol, $\beta$-sitosterol, stigmasterol, campesterol, $\alpha$-sitostanol, $\beta$-sitostanol, stigmastanol, campestanol, and cycloartenol, and esters thereof such as fatty acid esters, ferulic acid esters, and cinnamic acid esters.

In the pet food of the present invention, bran, chaffs, vegetables or vitamins may be blended. Examples of the bran include rice bran and wheat bran, and examples of the chaff include soybean chaff. Examples of the vegetables include a vegetable extract. Examples of the vitamins include A, B1, B2, D, E, niacin, pantothenic acid, and carotene. The content thereof is preferably 0.05 to 10%. In addition, the pet food may include additives which are generally used in a pet food, such as a gelling agent, a shape retaining agent, a pH adjuster, a seasoning, a preservative and a nutrition supplement. Further, a synthetic antioxidant such as BHA, BHT, ethoxyquin, TBHQ, or propyl gallate may also be included. Also in such a case, the technology of the present invention enables reduction in the amount of the synthetic antioxidant compared with ever before. It should be noted that the synthetic antioxidant is preferably not included in the pet food of the present invention.

The pet food of the present invention can be produced by mixing the above-mentioned components and shaping the mixture into a desired form, and the oil and fat component as the component (A) is preferably added finally. For example, citric acid and a citrate are mixed well together with cereal, meat meal, and mineral components such as iron and copper, and the resultant product is subjected to extrusion molding using an extruder while adding water or water vapor. After that, the resultant product is dried by hot air until the water content becomes preferably 10% or less. The oil and fat containing a large amount of a fatty acid having two or more double bonds is desirably coated after drying by hot air. In addition, the lecithin and catechin may be added together with citric acid or together with the oil and fat.

In addition, the pet food of the present invention may be any of dry-type, wet-type, semi-moist-type, jerky-type, biscuit-type, gum-type, granular, powdery, and soupy pet foods Of those, a dry-type pet food is preferred in view of easiness of storage. Usually, in the case of the dry-type pet food, the pet food for multiple feed is included in a laminate package. However, after opening the package, the pet food is easily brought into contact with air and hence is liable to be oxidized. Therefore, it can be said that, when the pet food of the present invention is a dry-type pet food, the effect is more exerted.

The dry-type pet food may have a kibble shape, a plate shape, or a born shape, for example. From the viewpoint of easiness of chewing by pets and an easily-handled shape, the pet food has a bulk density of preferably 100 to 900 kg/m$^3$, more preferably 300 to 700 kg/m$^3$. However, when the pet food has such bulk density, the pet food tends to have a large surface area and many voids. Therefore, it is considered that the oil and fat in the pet food is easily oxidized. However, even in such a case, the pet food according to the present invention has high antioxidative activity, and the above-mentioned advantages can be utilized. Therefore, the pet food has excellent palatability for pets, maintains the taste and flavor for a long period of time, and can provide benefits such as desired physiological effects due to the highly unsaturated fatty acid.

Examples

Test Example A

<Preparation of sample>

Various combinations of the (A) oil and fat, (B) mineral component, (C) citric acid+sodium citrate (mass ratio of 1:2; hereinafter, referred to as citric acid mixture), (D) lecithin, (E) polyphenol, and (X) another antioxidant shown in Table 1 were tested. The test was carried out by adding, to 3g of the (A) oil and fat, or a blend of the oil and fat with the (D) lecithin and (X) another antioxidant, dissolution of the (B) mineral component, (C) citric acid mixture, and (E) polyphenol in a small amount of water. It should be noted that each numerical value represented by "%" in Table 1 is "mass%" relative to the amount of the oil and fat.

[Table 1]

| Type | Symbol | Composition | Used amount |
|------|--------|-------------|-------------|
| A | a1 | Rapeseed oil+Fish oil | 3 g |
| | a2 | Rapeseed oil+Chicken oil | 3 g |

(continued)

| Type | Symbol | Composition | Used amount |
|---|---|---|---|
| B | b1 | Iron 0.01%+Copper 0.001% | 2.0 μmol |
| | b2 | Iron 0.03%+Copper 0.003% | 6.0 μmol |
| C | c1 | Citric acid mixture 0.33% | 13.3 μmol |
| | c2 | Citric acid mixture 0.50% | 20.0 μmol |
| | c3 | Citric acid mixture 1.00% | 40.0 μmol |
| | c4 | Citric acid mixture 3.00% | 120.0 μmol |
| D | d1 | Soybean lecithin | 0.30% |
| | d2 | Soybean lecithin | 0.60% |
| | d3 | Soybean lecithin | 3.00% |
| | d4 | Soybean-derived PE | 0.075% |
| | d5 | Soybean-derived PE | 0.10% |
| | d6 | Soybean-derived PE | 0.15% |
| | d7 | Soybean-derived PE | 0.60% |
| | d8 | Soybean-derived PE | 1.00% |
| | d9 | DPPE | 0.60% |
| | d10 | DOPE | 0.60% |
| | d11 | Soybean-derived PC | 0.60% |
| | d12 | DPPC | 0.60% |
| | d13 | Hydrogenated soybean lecithin | 0.60% |
| E | e1 | Catechin preparation | 0.30% |
| | e2 | Curcumin | 0.15% |
| | e3 | Quercetin | 0.05% |
| | e4 | Myricitrin | 0.05% |
| | e5 | Chlorogenic acid | 0.27% |
| | e6 | Proanthocyanidin | 0.95% |
| X | x1 | Mix tocopherol | 1.00% |
| | x2 | δ-Tocopherol | 1.00% |

Details of the materials used in the test are as follows.

(A) Oil and fat: The composition of fatty acids is as shown in Table 2.

(B) Mineral component

Ferric chloride: reagent, manufactured by Wako Pure Chemical Industries, Ltd.
Cupric chloride: reagent, manufactured by Wako Pure Chemical Industries, Ltd.

(C) Citric acid mixture

Sodiumcitrate (dihydrate) : reagent, manufactured by Wako Pure Chemical Industries, Ltd.
Citric acid (anhydrous): reagent, manufactured by Wako Pure Chemical Industries, Ltd.

(D) Lecithin

Lecithin (derived from soybeans): YELKIN TS (product name, manufactured by ADM Company, 63.3% in terms of phospholipid (active ingredient))
PE derived from soybeans: phosphatidylethanolamine (product name)
PC derived from soybeans: L-$\alpha$-phosphatidylcholine (product name, derived from soybeans; manufactured by Sigma-Aldrich Japan K.K.)
Hydrogenated soybean lecithin: L-$\alpha$-phosphatidylcholine (product name, manufactured by Wako Pure Chemical Industries, Ltd.)
DPPC: L-$\alpha$-phosphatidylcholine, dipalmitoyl (product name, manufactured by Wako Pure Chemical Industries, Ltd.)

(E) Polyphenol

Catechin preparation: polyphenone (product name, manufactured by Mitsui Norin Co., Ltd., tea extract, catechin content of 30%, and the composition of catechins is as shown in Table 3)

Curcumin: turmeric rhizome extract (product name, manufactured by YOKOHAMA OILS & FATS INDUSTRY CO., LTD., content of 15% or more)

Quercetin: onion skin/peel extract (product name, YOKOHAMA OILS & FATS INDUSTRY CO., LTD. content of 5.0 % or more)

Myricitrin: Sanmelin YO-1844 (product name, manufactured by San-Ei Gen F.F.I., Inc.)

(X) Another antioxidant

$\delta$-Tocopherol: manufactured by TAMA BIOCHEMICAL CO., LTD.

Mix-tocopherol: manufactured by Archer Daniels Midland Company

[Table 2]

| Fatty acid composition | A$_1$ Rapeseed oil | A$_2$ Fishoil | A$_3$ Chicken oil | a1: A$_1$+A$_2$ | a2: A$_1$+A$_3$ |
|---|---|---|---|---|---|
| C16 | 4.1 | 19.8 | 24.5 | 7.2 | 20.4 |
| C18:0 | 2.1 | 5.5 | 5.7 | 2.8 | 5.0 |
| C18:1 | 63.7 | 12.4 | 39.9 | 53.4 | 44.6 |
| C18:2 | 20.1 | 1.5 | 15.6 | 15.7 | 16.3 |
| C18:3 | 8.3 | 1.6 | 1.0 | 7.0 | 2.5 |
| EPA | - | 5.8 | - | 1.2 | - |
| DHA | - | 23.7 | - | 4.7 | - |
| Unsaturated fatty acid having two or more double bonds | 30.1 | 32.5 | 18.2 | 30.6 | 20.6 |
| Unsaturated fatty acid having three or more double bonds | 8.5 | 31.1 | 1.4 | 13.0 | 2.8 |

[Table 3]

| Catechins in catechin preparation | Content (%) |
|---|---|
| Catechin | 0.7 |

(continued)

| Catechins in catechin preparation | Content (%) |
|---|---|
| Epicatechin | 2.5 |
| Gallocatechin | 2.2 |
| Epigallocatechin | 9.4 |
| Epigallocatechin gallate | 13.0 |
| Gallocatechin gallate | 0.9 |
| Epicatechin gallate | 3.2 |
| Catechin gallate | 0.1 |
| Total | 32.0 |

<Method of evaluating antioxidant>

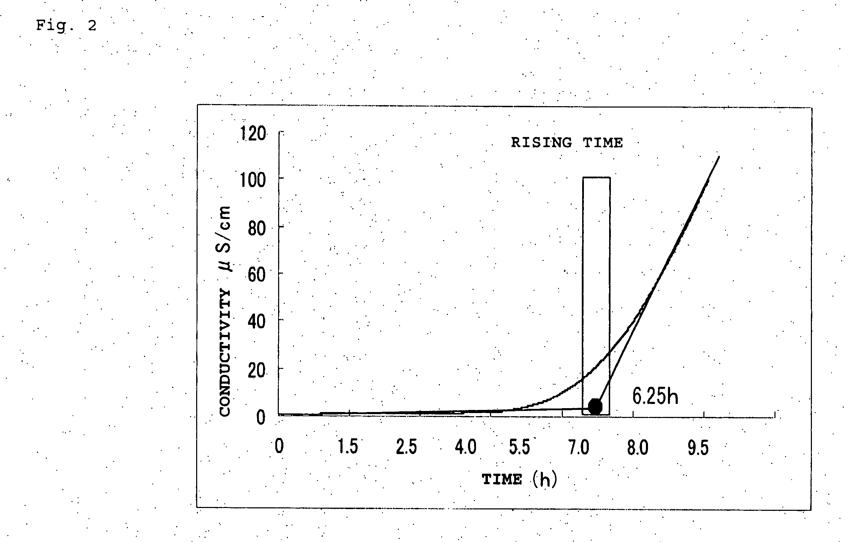

The prepared samples are placed in an automatic fat and oil stability test device ("Rancimat 743", manufactured by Metrohm) and maintained at 120°C while bubbling air under a condition of 20 mL/min, and the electrical conductivity is measured. The electrical conductivity is plotted with respect to the elapsed time, and the rising time (unit: hour) is calculated as shown in FIG. 2 to evaluate stability. This is an evaluation based on a principle that the electrical conductivity increases by oxidation, and a long rising time means that the sample has high resistance to oxidation.

Results of Test Example A

Table 4 shows the test results in the case where the (C) citric acid mixture, (D) lecithin, and (X) another oxidant were used as antioxidants, and Table 5 shows the test results in the case where the (E) polyphenol was further added.

[Table 4]

| Test number | A Oil and fat | B Mineral | C Citric acid mixture | D Lecithin | E Polyphenol | X Another oxidant | Rising time (h) |
|---|---|---|---|---|---|---|---|
| 1 | a1 | - | - | - | - | - | 2.2 |
| 2 | a1 | b1 | - | - | - | - | 0.5 |
| 3 | a1 | b1 | c1 | - | - | - | 2.2 |
| 4 | a1 | b1 | c2 | - | - | - | 2.2 |
| 5 | a1 | b1 | c3 | - | - | - | 2.2 |
| 6 | a1 | b1 | - | d2 | - | - | 1.5 |
| 7 | a1 | b1 | - | - | e1 | - | 0.8 |
| 8 | a1 | b1 | - | - | - | x1 | 0.5 |
| 9 | a1 | b1 | - | - | - | x2 | 0.5 |
| 10 | a1 | b1 | c3 | - | e1 | - | 2.1 |
| 11 | a1 | b1 | c3 | - | - | x1 | 1.0 |
| 12 | a1 | b1 | c3 | - | - | x2 | 1.4 |
| 13 | a1 | b1 | c1 | d2 | - | - | 1.0 |
| 14 | a1 | b1 | c1 | d3 | - | - | 1.3 |
| 15 | a1 | b1 | c2 | d2 | - | - | 2.7 |
| 16 | a1 | b1 | c2 | d3 | - | - | 4.0 |
| 17 | a1 | b1 | c3 | d1 | - | - | 3.3 |

(continued)

| Test number | A Oil and fat | B Mineral | C Citric acid mixture | D Lecithin | E Polyphenol | X Another oxidant | Rising time (h) |
|---|---|---|---|---|---|---|---|
| 18 | a1 | b1 | c3 | d2 | - | - | 4.6 |
| 19 | a1 | b1 | c3 | d4 | - | - | 2.5 |
| 20 | a1 | b1 | c3 | d5 | - | - | 3.1 |
| 21 | a1 | b1 | c3 | d6 | - | - | 5.7 |
| 22 | a1 | b1 | c3 | d7 | - | - | 14.2 |
| 23 | a1 | b1 | c3 | d8 | - | - | 21.6 |
| 24 | a1 | b1 | c3 | d9 | - | - | 8.5 |
| 25 | a1 | b1 | c3 | d10 | - | - | 20.0 |
| 26 | a1 | b1 | c3 | d11 | - | - | 2.7 |
| 27 | a1 | b1 | c3 | d12 | - | - | 6.0 |
| 28 | a1 | b1 | c3 | d13 | - | - | 5.9 |
| 29 | a1 | b2 | c3 | - | - | - | 2.2 |
| 30 | a1 | b2 | c3 | d2 | - | - | 1.5 |
| 31 | a1 | b2 | c4 | - | - | - | 2.2 |
| 32 | a1 | b2 | c4 | d2 | - | - | 3.3 |
| 33 | a2 | - | - | - | - | - | 11.9 |
| 34 | a2 | b1 | - | - | - | - | 0.7 |
| 35 | a2 | b1 | c3 | - | - | - | 6.7 |
| 36 | a2 | b1 | c3 | d2 | - | - | 4.6 |

[Table 5]

| Test number | A Oil and fat | B Mineral | C Citric acid mixture | D Lecithin | E Polyphenol | X Another oxidant | Rising time (h) |
|---|---|---|---|---|---|---|---|
| 5 | a1 | b1 | c3 | - | - | - | 2.2 |
| 18 | a1 | b1 | c3 | d2 | - | - | 4.6 |
| 37 | a1 | b1 | c3 | d2 | e1 | - | 12.0 |
| 38 | a1 | b1 | c3 | d2 | e2 | - | 5.0 |
| 39 | a1 | b1 | c3 | d2 | e3 | - | 5.2 |
| 40 | a1 | b1 | c3 | d2 | e4 | - | 6.8 |

<Discussion of Rancimat rising time>

The effect of the antioxidant was judged whether or not the rising time is extended compared with a composition before addition of the antioxidant. In addition, from past experience, the oil and fat used in the pet food was considered to have a Rancimat rising time (hereinafter, referred to as rising time) of preferably 3 hours or more. This was evaluated as a preferred composition.

<Comparison of oils and fats based on difference in fatty acid composition>

In the test numbers 1 to 32 and 37 to 40, the oil and fat a1 (containing an unsaturated fatty acid having three or more double bonds in an amount of 13.0%) was used, while in the test numbers 33 to 36, in order to compare evaluations of the antioxidative effects depending on the fatty acid compositions, a test was carried out using a2 (containing the same unsaturated fatty acid in an amount of 2.8%). In the cases of using only the oil and fat, the rising time of a1 was 2.2 hours, and the rising time of a2 was 11.9 hours, which suggested that there was a huge difference. However, when the mineral was further added, the rising time of a1 was 0.5 hour, and the rising time of a2 was 0.7 hour, which suggested that the antioxidative activity of a2 was significantly reduced to almost the same level of a1. When the citric acid mixture was added thereto, the rising time of a2 recovered to a sufficient level (6.7 hours) . However, the rising time of a1 recovered up to 2.2 hours, which was a level of addition of only the oil and fat. That is, in the case of the oil and fat containing the highly unsaturated fatty acid in a large amount, a technical ingenuity is required to achieve high antioxidative activity.

<Material capable of further enhancing effect of citric acid+citrate>

The pet foods of the test numbers 10 to 12 and 16 were studied. Each of the antioxidants was added to a composition including the oil and fat, mineral, and citric acid in an amount in terms of active ingredient shown in Table 4 (1.0% as is), and the results are shown. Only the lecithin (derived from soybeans) was found to have an effect of further enhancing the effect of the citric acid mixture.

<Amount of citric acid mixture>

Changes in the antioxidative activity were confirmed for the pet foods of test numbers 13 to 18 and 29 to 32 by changing the amount of the citric acid mixture relative to the amount of the mineral. In the case where the molar ratio to the mineral of the citric acid mixture (latter/former) was 6.7, the antioxidative activity was not improved and was found to tend to decrease compared with the case of addition of no lecithin. In the case where the molar ratio was 10, the effect of addition of the lecithinwas observed, and in the case where the lecithin was added in an amount of 3.0% relative to the oil and fat, the Rancimat time exceeded 3 hours, resulting in sufficient stability. In the case where the molar ratio was 20, a sufficient effect was obtained by adding lecithin in an amount of 0.6% relative to the oil and fat. When the mineral content was increased up to 3 times (test numbers 29 to 32), in the case where the molar ratio was 6.7, the effect obtained by addition of the lecithin was not observed, while in the case where the molar ratio was 20, the effect obtained by addition of the lecithin was observed. When the lecithin was added in an amount of 0.6% relative to the oil, sufficient results were obtained.

<With regard to kind and amount of lecithin>

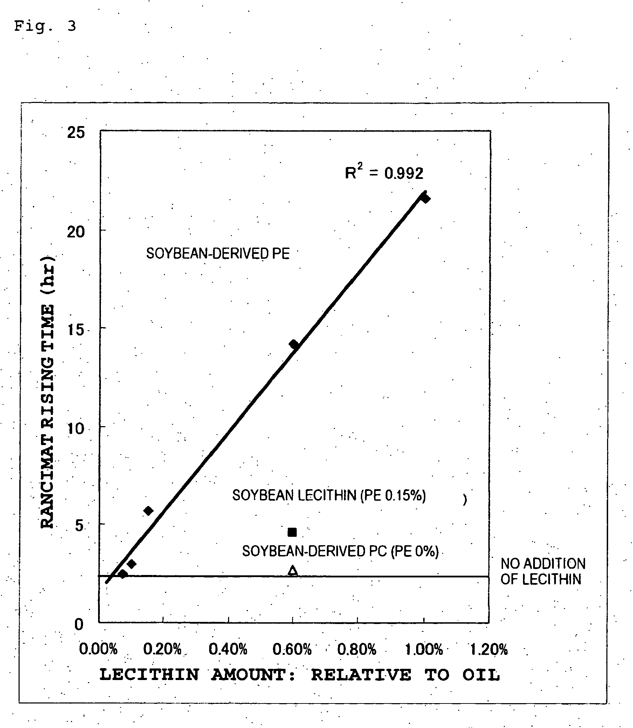

The amount of the lecithin which was able tobe used commercially or industrially in a pet food was determined based on acetone-insoluble matter of soybean lecithin. In addition, experiments using singly each component of soybean PC and soybean PE, which are glycerophospholipids of soybean lecithin, were carried out (test numbers 15 to 25). As a result, PE was found to have a higher activity compared with PC contained as a component in the largest amount. Further, the results of the test numbers 19 to 23 reveal that the antioxidative effect depends on the PE content (FIG. 3).

<Evaluation of polyphenol>

In the test numbers 37 to 40, the antioxidative activity was evaluated in the case where various polyphenols were added together with the above-mentioned citric acid mixture and lecithin. The catechins were found to extend the rising time and to drastically enhance the antioxidative activity. Also in the case of addition of curcumin, quercetin, or myricitrin, the antioxidative activity was improved. When the catechins were studied as a typical example (test numbers 7 and 10), the antioxidative activity was not improved by addition of only the catechins and by addition of the catechins together with the citric acid mixture (no lecithin). Therefore, the effect of improving the antioxidative activity is expressed only in a composition including all the citric acid, lecithin, and catechins.

Test Example B

<Preparation of sample>

Various combinations of the (A) oil and fat, (B) mineral component, (C) citric acid+sodium citrate (mass ratio of 1:2; hereinafter, referred to as citric acid mixture), (D) rosemary extract, and (X) another antioxidant shown in Table 6 were tested. The test was carried out by adding, to a blend of 3 g of the (A) oil and fat with the (D) rosemary extract and (X) another antioxidant, a dissolution of the (B) mineral component and (C) citric acid/citrate in a small amount of water.

[Table 6]

| Type | Symbol | Composition | Used amount |
|------|--------|-------------|-------------|
| A | a1 | Rapeseed oil+Fish oil (A1+A2) | 3 g |
| B | b1 | Iron 0.01%+Copper 0.001% | 2.0 μmol |
| C | c1 | Citric acid mixture 0.05% | 2.0 μmol |
| | c2 | Citric acid mixture 0.20% | 8.0 μmol |
| | c3 | Citric acid mixture 0.40% | 16.0 μmol |
| | c4 | Citric acid mixture 0.50% | 20.0 μmol |
| | c5 | Citric acid mixture 1.00% | 40.0 μmol |
| D | d1 | Rosemary extract (Herbalox) | 0.04% |
| | d2 | Rosemary extract (Herbalox) | 0.08% |
| | d3 | Rosemary extract (Herbalox) | 0.40% |
| | d4 | Rosemary extract (RM keeper) | 0.20% |
| X | x1 | Catechin preparation | 0.30% |
| | x2 | Catechin preparation | 0.60% |
| | x3 | Mix tocopherol | 1.00% |
| | x4 | $\delta$-Tocopherol | 1.00% |
| | x5 | Soybean lecithin | 0.60% |
| *The used amount of the rosemary extract is an amount in terms of extract. | | | |

It should be noted that details of the materials used for the test are shown below.

(A) Oil and fat: The composition of fatty acids is as shown in Table 2 above.
The (B) mineral component and (C) citric acid mixture are the same as those in Test Example A.
(D) Rosemary extract

Product name Herbalox HT-OC (rosemary extract content of 40%), manufactured by Kalsec Inc.

Product name RM keeper (rosemary extract content of 20%), manufactured by Mitsubishi-Kagaku Foods Corporation (X) Another antioxidant

Catechin preparation: product name polyphenone, Mitsui Norin Co., Ltd. (tea extract: content of 30%)

$\delta$-tocopherol: manufactured by TAMA BIOCHEMICAL CO.,LTD.

mix-tocopherol: manufactured by Archer Daniels Midland Company

[Table 7]

| | Numerical values in the table (%) | | |
|------|-------------|----------|-------------------|
| | Rapeseed oil | Fish oil | Rapeseed+Fish oil |
| C16:0 | 4.1 | 19.8 | 7.2 |
| C18:0 | 2.1 | 5.5 | 2.8 |
| C18:1 | 63.7 | 12.4 | 53.4 |

(continued)

| | Numerical values in the table (%) | | |
|---|---|---|---|
| | Rapeseed oil | Fish oil | Rapeseed+Fish oil |
| C18:2 | 20.1 | 1.5 | 15.7 |
| C18:3 | 8.3 | 1.6 | 7.0 |
| EPA | - | 5.8 | 1.2 |
| DHA | - | 23.7 | 4.7 |
| Unsaturated fatty acid having two or more double bonds | 30.1 | 32.5 | 30.6 |
| Unsaturated fatty acid having three or more double bonds | 8.5 | 31.1 | 13.0 |

<Method of evaluating antioxidant>

The method is the same as that in Test Example A.

Results of Test Example B

The results of Test Example B are shown in Table 8.

[Table 8]

| Test number | A Oil and fat | B Mineral | C Citric acid mixture | D Rosemary extract | X Other antioxidants | Rising time (h) |
|---|---|---|---|---|---|---|
| 41 | a1 | - | - | - | - | 2.2 |
| 42 | a1 | b1 | - | - | - | 0.5 |
| 43 | a1 | b1 | c1 | - | - | 0.8 |
| 44 | a1 | b1 | c5 | - | - | 2.2 |
| 45 | a1 | b1 | - | d3 | - | 0.5 |
| 46 | a1 | b1 | c1 | d2 | - | 0.7 |
| 47 | a1 | b1 | c2 | d2 | - | 2.6 |
| 48 | a1 | b1 | c3 | d2 | - | 3.0 |
| 49 | a1 | b1 | c4 | d2 | - | 3.5 |
| 50 | a1 | b1 | c5 | d1 | - | 2.6 |
| 51 | a1 | b1 | c5 | d2 | - | 3.0 |
| 52 | a1 | b1 | c5 | d3 | - | 5.3 |
| 53 | a1 | b1 | c4 | d3 | - | 5.5 |
| 54 | a1 | b1 | c5 | d4 | - | 3.4 |
| 55 | a1 | b1 | - | - | x1 | 0.8 |
| 56 | a1 | b1 | - | d3 | x1 | 1.4 |
| 57 | a1 | b1 | c5 | - | x1 | 2.1 |
| 58 | a1 | b1 | - | - | x2 | 1.5 |
| 59 | a1 | b1 | - | - | x3 | 0.5 |
| 60 | a1 | b1 | c5 | - | x3 | 1.1 |
| 61 | a1 | b1 | - | - | x4 | 0.5 |
| 62 | a1 | b1 | c5 | - | x4 | 1.0 |

(continued)

| Test number | A Oil and fat | B Mineral | C Citric acid mixture | D Rosemary extract | X Other antioxidants | Rising time (h) |
|---|---|---|---|---|---|---|
| 63 | a1 | b1 | c5 | d3 | x5 | 7.5 |

<Discussion of Rancimat rising time>

An evaluation was carried out in the same manner as in Test Example A. FIG. 4 shows a graph created based on a part of the results.

<Material capable of further enhancing effect of citric acid mixture>

The test numbers 52, 57, 60, and 62 are systems each including the oil and fat, mineral, and citric acid, and each composition contains each antioxidant added in an amount of 1.0 mass% as it is relative to the oil and fat and the results thereof are shown. As compared with the test number 44, only the rosemary extract was found to have an effect of further enhancing the effect of the citric acid mixture.

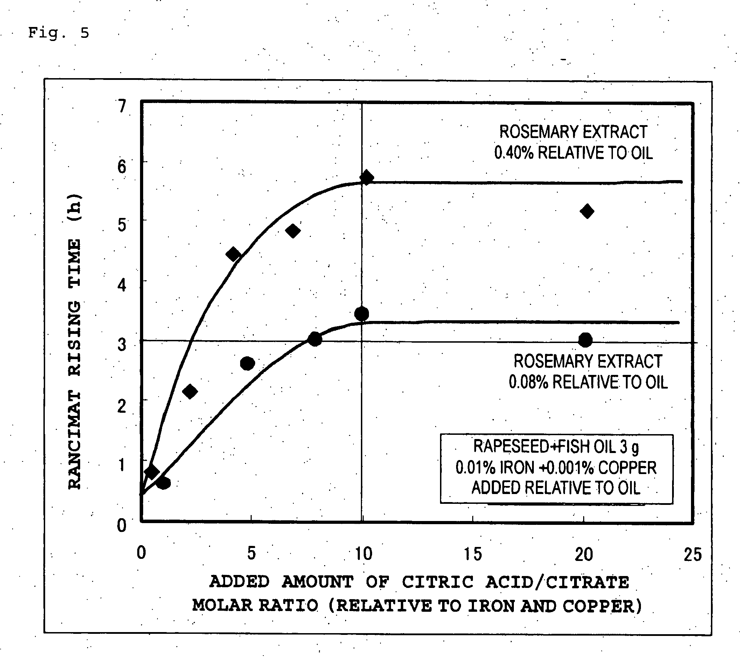

As compared with the citric acid mixture, the rosemary extract is generally expensive and has a characteristic odor. Therefore, in order to obtain the maximum effect by the minimum amount of the rosemary extract, it is necessary to adjust the amount of the citric acid mixture. The samples of the test numbers 45 to 49 and 51 were used to examine the concentration of the citric acid mixture, and FIG. 5 shows the results. It was found that when the citric acid mixture is added in an amount of at least 10 times by mol relative to the amount of the mineral, the oxidation-preventing effect in addition of the rosemary extract was stably obtained at a maximum.

In addition, when the concentration of the rosemary extract was changed in a composition in which the citric acid mixture was contained in an amount of at least 10 times by mol relative to the amount of the mineral, the stability in addition of the rosemary extract in an amount of 0.04 % exceeds that in addition of only the citric acid mixture. Therefore, the effect of addition of the rosemary extract was confirmed. In addition, when the rosemary extract was added in an amount of 0.08 % or more, oxidation stability sufficient as the pet food (rising time: more than 3 hours) was exhibited.

Further, in order to enhance the stability, the lecithin which was found to have a higheffect in Test Example A was used in combination (test number 63). The results revealed that the pet food had high stability.

<Evaluation of antioxidative activity of pet food (1) > Examples 1 and 2, Reference Example, and Comparative Examples 1 and 2

The raw materials having a composition shown in Table 9 were used and subjected to mixing extrusion molding using an extruder, to thereby produce a cylindrical pet food (kibbles) having a diameter of 10 mm and a length of about 10 mm. The pet food was found to have a bulk density of 472 kg/m$^3$.

Tables 10 to 12 show the composition of the glyceride or fatty acid in the oil and fat extracted from the pet food of Example 1, and the contents of the unsaturated fatty acid having two or more double bonds and the unsaturated fatty acid having three or more double bonds.

The content of all the oils and fats in the pet food was found to be 15%, the content of the unsaturated fatty acid having two or more double bonds in the pet food was found to be 27.2%, and the content of the unsaturated fatty acid having three or more double bonds was found to be 6.7%.

[Table 9]

| | | Example 1 | Example 2 | Reference Example | Comparative Example 1 | Comparative Example 2 |
|---|---|---|---|---|---|---|
| Pet food component | Barley | 27.00 | 27.00 | 27.00 | 27.00 | 27.00 |
| | Chicken meal | 23.00 | 23.00 | 23.00 | 23.00 | 23.00 |
| | Corn gluten | 12.00 | 12.00 | 12.00 | 12.00 | 12.00 |
| | Sorghum | 22.00 | 21.50 | 22.00 | 23.00 | 22.50 |
| | Rapeseed oil | 3.00 | 3.00 | 3.00 | 3.00 | 3.00 |
| | Fish oil | 2.00 | 2.00 | 2.00 | 2.00 | 2.00 |
| | Chicken oil | 3.75 | 3.75 | 4.00 | 4.00 | 4.00 |
| | Beet pulp | 3.00 | 3.00 | 3.00 | 3.00 | 3.00 |
| | Vitamin mineral mix | 3.00 | 3.00 | 3.00 | 3.00 | 3.00 |
| | Soybean lecithin (active ingredient 63.3%) | 0.25 | 0.25 | 0 | 0 | 0 |
| | Citric acid mixture A | 1.00 | 1.00 | 1.00 | 0 | 0 |
| | Catechin preparation | 0 | 0.50 | 0 | 0 | 0.50 |
| | Total | 100.00 | 100.00 | 100.00 | 100.00 | 100.00 |
| Analysis value | Iron (converted value) % | 0.015 | 0.015 | 0.015 | 0.015 | 0.015 |
| | Copper (converted value) % | 0.001 | 0.001 | 0.001 | 0.001 | 0.001 |
| | Citric acid (converted value) % | 1.09 | 1.09 | 1.09 | 0.13 | 0.13 |
| | Citric acid/(iron+copper) molar ratio | 15 | 15 | 15 | 2.37 | 2.37 |
| Evaluation | Increase in POV in oil and fat (16 weeks) | A | A | C | D | D |
| | Evaluation of odor | A | A | B | c | c |
| | pH of pet food | 5.3 | 5.4 | 5.4 | 5.6 | 5.6 |

[Table 10]

| Glyceride composition in extracted oil and fat | (wt%) |
|---|---|
| Fatty acid | 5.9 |
| Monoacylglycerol | 1.2 |
| Diacylglycerol | 2.9 |
| Triacylglycerol | 90 |
| Total | 100 |

[Table 11]

| Fatty acid composition in extracted oil and fat | (wt%) |
| --- | --- |
| C14:0 | 0.8 |
| C16:0 | 17 |
| C18:0 | 4.6 |
| C18:1 | 39.6 |
| C18:2 | 20.5 |
| C18:3 | 2.92 |
| EPA | 0.74 |
| DHA | 3.07 |
| Others | 10.77 |
| Total | 100 |

[Table 12]

| | (wt%) |
| --- | --- |
| Unsaturated fatty acid having two or more double bonds | 27.2 |
| Unsaturated fatty acid having three or more double bonds | 6.7 |

<Method of evaluating odor of oil and fat>

The pet foods were stored at room temperature (about 20°C) for 16 weeks, and changes in the odor of the oil and fat were evaluated by a sensory test by 5 humans. The pet food evaluated to "have little bad odor" by "4 or more humans" was represented as "A", the pet food evaluated to "have little bad odor" by "2 or 3 humans" was represented as "B", and the pet food evaluated to "have little bad odor" by "1 or less human" was represented as "C", in order of the degree of odor.

<Stability test method>

The pet foods were stored at room temperature (about 20°C), and at week 16, measurement and evaluation of the POVs (peroxide values) of the oil and fat in the pet foods were carried out by the following procedures.

<Method of measuring peroxide value (POV)>

The peroxide value (POV) was analyzed in accordance with 2.4.12.2-94 "Peroxide Value" in "Standard Methods for the Analysis of Fats, Oils and Related Materials" edited by Japan Oil Chemists' Society.

(1) Extraction of oil and fat from pet food

50 g of kibbles were pulverized and passed through a 1-mm mesh sieve, and a cylindrical filter paper was filled with the resultant sample and placed in an open column with a cock. With the cock kept closed, 150 mL of diethyl ether were added dropwise using a dropping funnel from the upper side. After completion of dropping, the cock of the column was opened to collect diethyl ether. Subsequently, the same procedure was carried out using 50 mL of diethyl ether. The collected diethyl ether was removed using an evaporator, to thereby obtain an extracted oil and fat from the pet food.

(2) Measurement of POV of oil and fat

0.5 g of the oil and fat extracted from the pet food was weighed in a 250-mL tall beaker and dissolved in 20 mL of a solution of acetic acid/isooctane=3/2 (volume ratio). 0.1 mL of a saturated potassium iodide solution was added to the tall beaker, and the mixture was shaken well for 1 minute. After that, 30 mL of distilled water were added to prepare a titration sample. The titration sample was titrated with 0.01 N sodium thiosulfate. In this procedure, an automatic POV titrator manufactured by METROHM-SHIBATA LTD., DMP Titrino type 785, was used.

(3) Evaluation of antioxidative effect

The pet food having an increase in POV of the oil and fat (16 weeks) of 5 or less was represented as "A", the pet food having an increase of more than 5 to 7 was represented as "B", the pet food having an increase of more than 7 to 9 was represented as "C", and the pet food having an increase of more than 9 was represented as "D". Table 9 shows the results. It should be noted that the pet food including no additive or including only the catechin was found to have an increase in POV of about 13.

<Method of measuring oxygen consumption>

As an index of an oxidation reaction of the pet food, oxygen consumption was measured in this test.

<Method of measuring converted value in terms of citric acid>

Kibbles (3.0g) werepulverizedbyamixer, 5 mL of 5% perchloric acid are added thereto, and the mixture is shaken for 10 minutes. After shaking, the volume is adjusted using a 50-mL measuring flask. The resultant solution is sonicated for 10 minutes and filtered, and the filtrate was subjected to measurement by high-performance liquid chromatography (HPLC). It should be noted that conditions of the HPLC measurement are as follows.
Column: Shodex Rspak KC-811, 2 columns, diameter 8 mmxlength 300 mm (manufactured by SHOWA DENKO K.K.)
Column temperature: 40°C
Mobile phase: 3 mmol/L perchloric acid aqueous solution
Reaction solution: 15 mmol/L disodium hydrogen phosphate aqueous solution containing 0.2 mmol/L bromthymol blue
Flow volume: 1.0 mL/min for mobile phase, 1.4 mL/min for reaction solution
Measurement wavelength: 445 nm

<Method of measuring PE amount (converted value in terms of phospholipid)>

An oil and fat was extracted from the pet food with a solvent of chloroform:methanol=2:1. The content of phosphorous in the extracted oil and fat was quantified. The content of phosphorous in the oil and fat was quantified by a phospholipid colorimetric method described in 2.4.11-1996 in "Standard Methods for the Analysis of Fats, Oils and Related Materials" edited by Japan Oil Chemists' Society (conversion factor from phosphorus level: 25.4). The content in the pet food was calculated by multiplying the content in the oil and fat by the content of the oil and fat in the pet food.
The percentage of PE in the phospholipid was determined in accordance with Phospholipid Composition (thin-layer chromatography) Standard Methods for the Analysis of Fats, Oils and Related Materials 4.3.3.1-1996, and multiplied by the above-mentioned converted value in terms of phospholipid to determine the concentration of PE.
The results shown in Table 9 suggest that stability was significantly improved in the case of using the two components including the citric acid and lecithin and in the case of using the three components including the citric acid, lecithin, and catechin. It should be noted that the pet foods of Examples 1 and 2 had a PE content of 0.04%.

<Evaluation of antioxidative activity in pet food (2) > Examples 3 and 4 and Comparative Example 3

The raw materials shown in Table 13 were used and subjected to mixing extrusion molding using an extruder, to thereby produce a cylindrical pet food (kibbles) having a diameter of 8.5 mm and a length of about 5.5 mm. The pet food was found to have a bulk density of 428 kg/m$^3$.
Tables 11 and 12 show the compositions of glycerides and fatty acids in the oil and fat extracted from the pet food of Example 3 and the contents of unsaturated fatty acids having two or more and three or more double bonds.
The content of all the oils and fats in the pet food was 15%, the content of the unsaturated fatty acid having two or more double bonds in the pet food was 27.2%, and the content of the unsaturated fatty acid having three or more double bonds in the pet food was 6.7%.

[Table 13]

| | | Example 3 | Example 4 | Comparative Example 3 |
|---|---|---|---|---|
| Components of pet food | Corn | 27.00 | 27.00 | 27.00 |
| | Chicken meal | 23.00 | 23.00 | 23.00 |
| | Corn gluten | 12.00 | 12.00 | 12.00 |
| | Sorghum | 21.50 | 21.50 | 22.50 |
| | Soybean oil | 4.00 | 4.00 | 4.00 |
| | Linseed oil | 1.50 | 1.50 | 1.50 |
| | Chicken oil | 3.50 | 3.75 | 3.75 |
| | Beet pulp | 3.00 | 3.00 | 3.00 |
| | Vitamin mineral mix | 3.00 | 3.00 | 3.00 |
| | Soybean lecithin (active ingredient 63.3%) | 0.50 | 0.25 | 0.25 |
| | Citric acid mixture A | 1.00 | 1.00 | 0 |
| | Total | 100.00 | 100.00 | 100.00 |
| Analysis value | Iron (converted value) % | 0.0136 | 0.0136 | 0.015 |
| | Copper (converted value) % | 0.0014 | 0.0014 | 0.001 |
| | Citric acid (converted value) % | 0.94 | 0.94 | 0.13 |
| | Citric acid/(iron+copper) molar ratio | 18.5 | 18.5 | 2.37 |
| | PE | 0.08 | 0.04 | 0.04 |
| Accelerated evaluation | Increase in POV of oil and fat (40°C, 8 weeks) | A | B | D |
| | Evaluation of odor | A | B | c |
| | Slope of oxygen consumption mL/week | 1.02 | 1.5 | 1.9 |
| | pH of pet food | 5.3 | 5.3 | 5.6 |

[Table 14]

| Fatty acid composition in extracted oil and fat | (wt%) |
|---|---|
| C14:0 | 0.4 |
| C16:0 | 16 |
| C18:0 | 5.1 |
| C18:1 | 32.5 |
| C18:2 | 33.9 |
| C18:3 | 8.0 |
| EPA | 0 |
| DHA | 0 |

[Table 15]

| | (wt%) |
|---|---|
| Unsaturated fatty acid having two or more double bonds | 41.9 |
| Unsaturated fatty acid having three or more double bonds | 8.0 |

<Method of evaluating odor of oil and fat>

The pet foods were stored for 8 weeks at 40°C, and changes in the odor of the oil and fat were evaluated by a sensory test by 5 humans. The pet food evaluated to "have little bad odor" by "4 or more humans" was represented by "A", the pet food evaluated to "have little bad odor" by "2 or 3 humans" was represented by "B", and the pet food evaluated to "have little bad odor" by "1 or less human" was represented by "C", in order of the degree of odor.

(3) Evaluation of antioxidative effect

The pet food having an increase in POV of the oil and fat (40°C, 8 weeks) of 5 or less was represented by "A", the pet food having an increase of more than 5 and less than 7 was represented by "B", the pet food having an increase of more than 7 and less than 10 was represented by "C", and the pet food having an increase of more than 10 was represented by "D". Table 13 shows the results.

<Method of quantifying oxidation stability>

As an index of oxidation stability of the pet food, the POV value after storage has been used. The value was obtained by allowing oxygen to react with the double bond moiety in the oil and fat to produce a peroxide and quantifying the peroxide. The value is a very good index to examine deterioration of the oil and fat, and the method is the most general. However, it takes much time to measure an increase in POV, i.e., at least two months in storage at 40°C as an accelerated test or 4 months at room temperature, and there is a problem with the long time. In addition, the peroxide is not a final product in oxidation, and is highly reactive, turns into a carbonyl group and then a carboxyl group by autoxidat ion. Moreover, there arises a problem in that POV is not changed or decreases in spite of clear progress of the oxidation reaction particularly at high temperature. Therefore, in the present invention, the total oxidation reaction was evaluated by measuring the amount of decreased oxygen as an index of the oxidation reaction.

<Method of measuring oxygen consumption>

50 g of the pet food were weighed, hermetically sealed with a packaging material including laminated aluminum, and stored in an incubator at 40°C. The oxygen concentration $X_t$ (%) in air in the packaging material was measured every 1 week to calculate an oxygen decrease rate. The initial air volume V (mL) in the packaging material was measured, and the oxygen consumption per 50 g of the pet food was calculated by the following equation.

$$\text{Oxygen consumption (mL)} = V \cdot (20.8 - X_t)/100$$

The relationship between the storage period and the oxygen consumption was found to be a very good linear relationship, and the slope of the line was used as an index of stability of the pet food.
Also in the pet food having a high polyunsaturated fatty acid content, stability was significantly improved by blending the citric acid and lecithin. In addition, the test revealed that, in the case where only the lecithin was blended without blending citric acid, stability was insufficient.
The oxygen consumption is effectively used as an index of stability. The oxygen consumption was evaluated based on POV and the results of sensory evaluation, and the pet food having a value of less than 1.2 was represented by "A", the pet food having a value of 1.2 or more and less than 1.6 was represented by "B", the pet food having a value of 1.6 or more and less than 2.0 was represented by "C", and the pet food having a value of 2.0 or more was represented by "D".

<Evaluation of antioxidative activity in pet food (3) >

The raw materials having the composition shown in Table 16 were used and subj ected to mixing extrusion molding using an extruder, to thereby produce a cylindrical pet food (kibbles) having a diameter of 10 mm and a length of about 10 mm. The pet food was found to have a bulk density of 472 kg/m$^3$.

Tables 17 to 19 show the compositions of the glycerides and fatty acids in the oil and fat extracted from the pet food of Reference Example, and the contents of the unsaturated fatty acid having two or more double bonds and the unsaturated fatty acid having three or more double bonds.

The content of all the oils and fats in the pet food was found to be 15%, the content of the unsaturated fatty acid having two or more double bonds in the pet food was found to be 28.2%, and the content of the unsaturated fatty acid having three or more double bonds was found to be 7.45%.

[Table 16]

| Composition of pet food (mass%) | Composition 1 | Composition 2 | Composition 3 |
|---|---|---|---|
| Antioxidation system | Only citric acid mixture A | Citric acid mixture A+rosemary extract | |
| | Reference Example | Example | Comparative Example |
| Barley | 27 | 27 | 27 |
| Chicken meal | 23 | 23 | 23 |
| Corn gluten meal | 12 | 12 | 12 |
| Sorghum (red milo) | 22 | 21.975 | 23 |
| Rapeseed oil | 3 | 3 | 3 |
| Fish oil | 2 | 2 | 2 |
| Rosemary (Herbalox) | 0 | 0.025 | 0 |
| Beet pulp | 3 | 3 | 3 |
| Chicken oil | 4 | 4 | 4 |
| Vitamin mineral mix | 3 | 3 | 3 |
| Citric acid mixture (in terms of citric acid) | 1 (1.09) | 1 (1.09) | 0 (0.13) |
| Total | 100 | 100 | 100 |
| Iron (converted value) | 0.015 | 0.015 | 0.015 |
| Copper (converted value) | 0.001 | 0.001 | 0.001 |
| Molar ratio of citric acid to iron and copper | 15 | 15 | 1.8 |
| Increase in POV of oil and fat (4 weeks) | B | A | c |
| Evaluation of odor of pet food | B | A | c |
| pH of pet food | 5.4 | 5.3 | 5.6 |

[Table 17]

Glyceride composition in extracted oil and fat (mass%)

| Free fatty acid | 5.6 |
|---|---|
| Monoacylglycerol | 1.0 |
| Diacylglycerol | 2.4 |
| Triacylglycerol | 91 |
| Total | 100 |

[Table 18]

| Fatty acid composition in extracted oil and fat (mass%) | |
|---|---|
| C14:0 | 0.9 |
| C16:0 | 15.7 |
| C18:0 | 4 |
| C18:1 | 39.8 |
| C18:2 | 20.7 |
| C18:3 | 3.1 |
| EPA | 0.9 |
| DHA | 3.5 |
| Others | 11.4 |
| Total | 100 |

[Table 19]

| | (mass%) |
|---|---|
| Unsaturated fatty acid having two or more double bonds | 28.2 |
| Unsaturated fatty acid having three or more double bonds | 7.5 |

<Method of evaluating odor of oil and fat>

The pet foods were stored for 4 weeks at room temperature (about 20°C), and changes in the odor of the oil and fat were evaluated by a sensory test by 5 humans. The pet food evaluated to "have little bad odor" by "4 or more humans" was represented by "A", the pet food evaluated to "have little bad odor" by "2 or 3 humans" was represented by "B", and the pet food evaluated to "have little bad odor" by "1 or less human" was represented by "C", in order of the degree of odor.

<Method of stability test>

The pet foods were stored at room temperature (about 20°C) and sampled at week 0 and at week 4, and the POVs (peroxide values) of the oil and fat in the pet foods were measured. Table 16 shows increases in the POVs during the period.

<Method of measuring peroxide value (POV)>

The peroxide value (POV) was analyzed in accordance with 2.4.12.2-94 "Peroxide Value" in "Standard Methods for the Analysis of Fats, Oils and Related Materials" edited by Japan Oil Chemists' Society.

(1) Extraction of oil and fat from pet food

50 g of kibbles were pulverized and passed through a 1-mm mesh sieve, and a cylindrical filter paper was filled with the resultant sample and placed in an open column with a cock. With the cock kept closed, 150 mL of diethyl ether was added dropwise using a dropping funnel from the upper side. After completion of dropping, the cock of the column was opened to collect diethyl ether. Subsequently, the same procedure was carried out using 50 mL of diethyl ether. The collected diethyl ether was removed using an evaporator, to thereby obtain an extracted oil and fat from the pet food.

(2) Measurement of POV of oil and fat

0.5 g of the oil and fat extracted from the pet food was weighed in a 250 mL tall beaker, and dissolved in 20 mL of a solution of acetic acid/isooctane=3/2 (volume ratio). 0.1 mL of a saturated potassium iodide solution was added to the tall beaker, and the mixture was shaken well for 1 minute. After that, 30 mL of distilled water was added thereto to prepare a titration sample. The titration sample was titrated with 0.01 N sodium thiosulfate. In this experiment, an automatic POV titrator manufactured by METROHM-SHIBATA LTD., DMP Titrino type 785, was used.

(3) Evaluation of antioxidative effect

The pet food having an increase in POV in the oil and fat (4 weeks) of 10 or less was represented by "A", the pet food having an increase of more than 10 and 15 or less was represented by "B", and the pet food having an increase of more than 15 was represented by "C". Table 16 shows the results. It should be noted that the pet food including no additive was found to have a POV of about 35.

<Method of measuring converted value in terms of citric acid>

Kibbles (3.0 g) werepulverizedbyamixer, 5mL of 5% perchloric acid were added thereto, and the mixture was shaken for 10 minutes. After shaking, the volume of the mixture was adjusted using a 50 mL measuring flask. The mixture was sonicated for 10 minutes and filtered, and the filtrate was subjected to measurement by high-performance liquid chromatography (HPLC). It should be noted that conditions of HPLC measurement are as follows.
Column: Shodex Rspak KC-811, 2 columns, diameter 8 mmxlength 300 mm (manufactured by SHOWA DENKO K.K.)
Column temperature: 40°C
Mobile phase: 3 mmol/L perchloric acid aqueous solution
Reaction solution: 15 mmol/L disodium hydrogen phosphate aqueous solution containing 0.2 mmol/L bromthymol blue
Flow volume: 1.0 mL/min for mobile phase, 1.4 mL/min for reaction solution
Measurement wavelength: 445 nm
As shown in Table 16, the pet food of Example was found to have high oxidation stability in 4-week storage.

## Claims

1. A pet food, comprising the following components (A) to (D) :

   (A) an oil and fat comprising, as a constituent fatty acid, an unsaturated fatty acid having two or more double bonds;
   (B) a mineral component selected from iron, an iron compound, copper, and a copper compound;
   (C) one or more kinds selected from citric acid and a salt thereof; and
   (D) one or more kinds selected from a lecithin and a rosemary extract,

   wherein a total amount of the component (C) is 10 times or more that of the component (B) in terms of molar ratio.

2. The pet food according to claim 1, wherein the component (D) comprises any one of the lecithin and the rosemary extract.

3. The pet food according to claim 1, wherein the component (D) comprises both of the lecithin and the rosemary extract.

4. The pet food according to any one of claims 1 to .3, wherein the component (A) is an oil and fat comprising, as a constituent fatty acid, 20 to 90 mass% of an unsaturated fatty acid having two or more double bonds.

5. The pet food according to any one of claims 1 to 4, wherein the component (A) is an oil and fat comprising, as a constituent fatty acid, 3 mass% or more of an unsaturated fatty acid having three or more double bonds.

6. The pet food according to any one of claims 1 to 5, wherein a content of the component (A) in the pet food is 5 to 80 mass%.

7. The pet food according to any one of claims 1 to 6, wherein the mineral component (B) is comprised as iron or an iron compound in an amount of 0.00001 to 1 mass% in terms of iron in the pet food.

8. The pet food according to any one of claims 1 to 7, wherein the mineral component (B) is comprised as copper or a copper compound in an amount of 0.00001 to 0.1 mass% in the pet food.

9. The pet food according to any one of claims 1 to 8, wherein the component (C) is one or more kinds selected from citric acid and an alkali metal citrate.

10. The pet food according to any one of claims 1 to 9, wherein the total amount of the component (C) is 10 to 60 times that of the component (B) in terms of molar ratio.

11. The pet food according to any one of claims 1 to 10, wherein the component (D) comprises phosphatidylethanolamine, and a content of the phosphatidylethanolamine in the pet food is 0.03 mass% or more.

12. The pet food according to any one of claims 1 to 11, wherein a content of the lecithin as the component (D) in the pet food is 0.06 to 6.0 mass%.

13. The pet food according to any one of claims 1 to 12, wherein a content of the rosemary extract as the component (D) in the pet food is 0.004 mass% or more.

14. The pet food according to any one of claims 1 to 13, further comprising (E) at least one kind of polyphenol selected from catechins, curcumin, quercetin, and myricitrin.

15. The pet food according to claim 14, wherein the component (E) is catechins.

16. The pet food according to claim 14 or 15, wherein a content of the component (E) in the pet food is 0.001 to 35 mass%.

17. The pet food according to any one of claims 1 to 16, wherein the pet food has a pH of 3.5 to 7.0.

18. The pet food according to any one of claims 1 to 17, wherein the pet food is dry-type.

19. A method of preventing oxidation of a composition comprising (A) an oil and fat comprising, as a constituent fatty acid, an unsaturated fatty acid having two or more double bonds and (B) a mineral component selected from iron, an iron compound, copper, and a copper compound,
the method comprising blending, in the composition, (C) one or more kinds selected from citric acid and a salt thereof and (D) one or more kinds selected from a lecithin and a rosemary extract,
wherein a total amount of the components (C) is 10 times or more that of the component (B) in terms of molar ratio.

20. The method of preventing oxidation according to claim 19, further comprising blending the composition with a plant extract comprising (E) at least one kind of polyphenol selected from catechins, curcumin, quercetin, and myricitrin.

**Patentansprüche**

1. Eine Haustiernahrung, umfassend die folgenden Bestandteile (A) bis (D):

   (A) ein Öl und Fett, umfassend, als eine konstituierende Fettsäure, eine ungesättigte Fettsäure mit zwei oder mehr Doppelbindungen;
   (B) einen mineralischen Bestandteil, ausgewählt aus Eisen, einer Eisenverbindung, Kupfer und einer Kupferverbindung;
   (C) eine oder mehrere Arten, ausgewählt aus Citronensäure und einem Salz davon; und
   (D) eine oder mehrere Arten, ausgewählt aus einem Lecithin und einem Rosmarinextrakt,

   wobei eine Gesamtmenge des Bestandteils (C), ausgedrückt als Molverhältnis, um das 10-Fache oder mehr höher ist als die des Bestandteils (B).

2. Die Haustiernahrung nach Anspruch 1, wobei der Bestandteil (D) eines aus dem Lecithin und dem Rosmarinextrakt umfasst.

3. Die Haustiernahrung nach Anspruch 1, wobei der Bestandteil (D) sowohl das Lecithin als auch den Rosmarinextrakt umfasst.

4. Die Haustiernahrung nach einem der Ansprüche 1 bis 3, wobei der Bestandteil (A) ein Öl und Fett ist, das, als konstituierende Fettsäure, 20 bis 90 Masse-% einer ungesättigten Fettsäure mit zwei oder mehr Doppelbindungen umfasst.

5. Die Haustiernahrung nach einem der Ansprüche 1 bis 4, wobei der Bestandteil (A) ein Öl und Fett ist, das, als konstituierende Fettsäure, 3 Masse-% oder mehr einer ungesättigten Fettsäure mit drei oder mehr Doppelbindungen umfasst.

6. Die Haustiernahrung nach einem der Ansprüche 1 bis 5, wobei ein Gehalt des Bestandteils (A) in der Haustiernahrung 5 bis 80 Masse-% beträgt.

7. Die Haustiernahrung nach einem der Ansprüche 1 bis 6, wobei der mineralische Bestandteil (B) als Eisen oder eine Eisenverbindung in einer Menge von 0,00001 bis 1 Masse-%, ausgedrückt als Eisen, in der Haustiernahrung enthalten ist.

8. Die Haustiernahrung nach einem der Ansprüche 1 bis 7, wobei der mineralische Bestandteil (B) als Kupfer oder eine Kupferverbindung in einer Menge von 0,00001 bis 0,1 Masse-% in der Haustiernahrung enthalten ist.

9. Die Haustiernahrung nach einem der Ansprüche 1 bis 8, wobei der Bestandteil (C) eine oder mehrere Arten, ausgewählt aus Citronensäure und einem Alkalimetallcitrat, ist.

10. Die Haustiernahrung nach einem der Ansprüche 1 bis 9, wobei die Gesamtmenge des Bestandteils (C), ausgedrückt als Molverhältnis, das 10- bis 60-Fache derer des Bestandteils (B) beträgt.

11. Die Haustiernahrung nach einem der Ansprüche 1 bis 10, wobei der Bestandteil (D) Phosphatidylethanolamin umfasst und ein Gehalt des Phosphatidylethanolamins in der Haustiernahrung 0,03 Masse-% oder mehr beträgt.

12. Die Haustiernahrung nach einem der Ansprüche 1 bis 11, wobei ein Gehalt des Lecithins als der Bestandteil (D) in der Haustiernahrung 0,06 bis 6,0 Masse-% beträgt.

13. Die Haustiernahrung nach einem der Ansprüche 1 bis 12, wobei ein Gehalt des Rosmarinextrakts als der Bestandteil (D) in der Haustiernahrung 0,004 Masse-% oder mehr beträgt.

14. Die Haustiernahrung nach einem der Ansprüche 1 bis 13, weiter umfassend (E) mindestens eine Art von Polyphenol, ausgewählt aus Catechinen, Curcumin, Quercetin und Myricitrin.

15. Die Haustiernahrung nach Anspruch 14, wobei der Bestandteil (E) Catechine ist.

16. Die Haustiernahrung nach Anspruch 14 oder 15, wobei ein Gehalt des Bestandteils (E) in der Haustiernahrung 0,001 bis 35 Masse-% beträgt.

17. Die Haustiernahrung nach einem der Ansprüche 1 bis 16, wobei die Haustiernahrung einen pH-Wert von 3,5 bis 7,0 aufweist.

18. Die Haustiernahrung nach einem der Ansprüche 1 bis 17, wobei die Haustiernahrung vom trockenen Typ ist.

19. Ein Verfahren zur Verhinderung der Oxidation einer Zusammensetzung umfassend (A) ein Öl und Fett, umfassend, als eine konstituierende Fettsäure, eine ungesättigte Fettsäure mit zwei oder mehr Doppelbindungen und (B) einen mineralischen Bestandteil, ausgewählt aus Eisen, einer Eisenverbindung, Kupfer und einer Kupferverbindung, wobei das Verfahren das Beimischen von (C) einer oder mehreren Arten, ausgewählt aus Citronensäure und einem Salz davon, und (D) einer oder mehreren Arten, ausgewählt aus einem Lecithin und einem Rosmarinextrakt, in die Zusammensetzung umfasst, wobei eine Gesamtmenge des Bestandteils (C), ausgedrückt als Molverhältnis, um das 10-Fache oder mehr höher ist als die des Bestandteils (B).

20. Das Verfahren zur Verhinderung der Oxidation nach Anspruch 19, weiter umfassend Mischen der Zusammensetzung mit einem Pflanzenextrakt, umfassend (E) mindestens eine Art von Polyphenol, ausgewählt aus Catechinen, Curcumin, Quercetin und Myricitrin.

**Revendications**

1. Aliment pour animal de compagnie, comprenant les composants (A) à (D) suivants :

    (A) une huile et matière grasse comprenant, à titre d'acide gras constitutif, un acide gras insaturé ayant deux doubles liaisons ou plus ;
    (B) un composant minéral choisi parmi le fer, un composé de fer, le cuivre, et un composé de cuivre ;
    (C) un composant d'un ou de plusieurs types choisis parmi l'acide citrique et un de ses sels ; et
    (D) un composant d'un ou de plusieurs types choisis parmi une lécithine et un extrait de romarin,

    dans lequel une quantité totale du composant (C) est 10 fois ou plus celle du composant (B) en termes de rapport molaire.

2. Aliment pour animal de compagnie selon la revendication 1, dans lequel le composant (D) comprend tout type de lécithine ou extrait de romarin.

3. Aliment pour animal de compagnie selon la revendication 1, dans lequel le composant (D) comprend à la fois la lécithine et l'extrait de romarin.

4. Aliment pour animal de compagnie selon l'une quelconque des revendications 1 à 3, dans lequel le composant (A) est une huile et matière grasse comprenant, à titre d'acide gras constitutif, 20 à 90 % en poids d'un acide gras insaturé ayant deux doubles liaisons ou plus.

5. Aliment pour animal de compagnie selon l'une quelconque des revendications 1 à 4, dans lequel le composant (A) est une huile et matière grasse comprenant, à titre d'acide gras constitutif, 3 % en poids ou plus d'un acide gras insaturé ayant trois doubles liaisons ou plus.

6. Aliment pour animal de compagnie selon l'une quelconque des revendications 1 à 5, dans lequel une teneur du composant (A) dans l'aliment pour animal de compagnie est de 5 à 80 % en poids.

7. Aliment pour animal de compagnie selon l'une quelconque des revendications 1 à 6, dans lequel le composant minéral (B) est compris sous forme de fer ou de composé de fer en une quantité de 0,00001 à 1 % en poids en termes de fer dans l'aliment pour animal de compagnie.

8. Aliment pour animal de compagnie selon l'une quelconque des revendications 1 à 7, dans lequel le composant minéral (B) est compris sous forme de cuivre ou de composé de cuivre en une quantité de 0,00001 à 0,1 % en poids dans l'aliment pour animal de compagnie.

9. Aliment pour animal de compagnie selon l'une quelconque des revendications 1 à 8, dans lequel le composant (C) est d'un ou de plusieurs types choisis parmi l'acide citrique et un citrate de métal alcalin.

10. Aliment pour animal de compagnie selon l'une quelconque des revendications 1 à 9, dans lequel la quantité totale du composant (C) est 10 à 60 fois celle du composant (B) en termes de rapport molaire.

11. Aliment pour animal de compagnie selon l'une quelconque des revendications 1 à 10, dans lequel le composant (D) comprend une phosphatidyléthanolamine, et une teneur de phosphatidyléthanolamine dans l'aliment pour animal de compagnie est de 0,03 % en poids ou plus.

12. Aliment pour animal de compagnie selon l'une quelconque des revendications 1 à 11, dans lequel une teneur de lécithine à titre de composant (D) dans l'aliment pour animal de compagnie est de 0,06 à 6,0 % en poids.

13. Aliment pour animal de compagnie selon l'une quelconque des revendications 1 à 12, dans lequel une teneur de l'extrait de romarin à titre de composant (D) dans l'aliment pour animal de compagnie est de 0,004 % en poids ou plus.

14. Aliment pour animal de compagnie selon l'une quelconque des revendications 1 à 13, comprenant en outre (E) au moins un type de polyphénol choisi parmi les catéchines, la curcumine, la quercétine, et la myricitrine.

15. Aliment pour animal de compagnie selon la revendication 14, dans lequel le composant (E) est des catéchines.

**16.** Aliment pour animal de compagnie selon la revendication 14 ou 15, dans lequel une teneur du composant (E) dans l'aliment pour animal de compagnie est de 0,001 à 35 % en poids.

**17.** Aliment pour animal de compagnie selon l'une quelconque des revendications 1 à 16, dans lequel l'aliment pour animal de compagnie a un pH de 3,5 à 7,0.

**18.** Aliment pour animal de compagnie selon l'une quelconque des revendications 1 à 17, dans lequel l'aliment pour animal de compagnie est de type sec.

**19.** Méthode de prévention de l'oxydation d'une composition comprenant (A) une huile et matière grasse comprenant, à titre d'acide gras constitutif, un acide gras insaturé ayant deux doubles liaisons ou plus et (B) un composant minéral choisi parmi le fer, un composé de fer, le cuivre, et un composé de cuivre, la méthode comprenant le mélange, dans la composition, (C) d'un composant d'un ou de plusieurs types choisis parmi l'acide citrique et un de ses sels et (D) un composant d'un ou de plusieurs types choisis parmi une lécithine et un extrait de romarin, dans laquelle une quantité totale du composant (C) est 10 fois ou plus celle du composant (B) en termes de rapport molaire.

**20.** Méthode de prévention de l'oxydation selon la revendication 19, comprenant en outre le mélange de la composition avec un extrait végétal comprenant (E) au moins un type de polyphénol choisi parmi les catéchines, la curcumine, la quercétine, et la myricitrine.

Fig.1

Fig. 2

Fig. 3

Figure 3 — Graph of RANCIMAT RISING TIME (hr) versus LECITHIN AMOUNT: RELATIVE TO OIL. $R^2 = 0.992$. Data series labeled: SOYBEAN-DERIVED PE, SOYBEAN LECITHIN (PE 0.15%), SOYBEAN-DERIVED PC (PE 0%), NO ADDITION OF LECITHIN.

Fig. 4

EP 2 578 087 B1

Fig. 5

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2005204659 A
- JP 2007110916 A
- JP 2004115758 A
- JP 2007185138 A
- JP 7059548 A

**Non-patent literature cited in the description**

- Standard Methods for the Analysis of Fats, OilsandRelatedMaterials, 5.3.3.1-86 Phospholipid Phosphorus Composition
- Standard Methods for the Analysis of Fats, Oils and Related Materials. Japan Oil Chemists' Society, 02 November 1996
- Peroxide Value. Standard Methods for the Analysis of Fats, Oils and Related Materials. 02 December 1994
- Standard Methods for the Analysis of Fats, Oils and Related Materials. 02 November 1996